# EUROPEAN PATENT APPLICATION

(11) **EP 1 609 486 A1**
(43) Date of publication of application: **28.12.2005**
(21) Application number: 05105198.5
(22) Date of filing: 14.06.2005
(51) Int. Cl.: A61L 2/18, A01K 1/035

(54) **Device and method for isolating contaminated bodies and materials**

(30) Priority: 14.06.2004 IT MI20041185
(71) Applicant: TECNIPLAST S.p.A., 21020 Buguggiate, Varese (IT)
(72) Inventor: Tartaglia, Giovanni, 28066, Galliate (IT); Campiotti, Alfredo, 21046, Malnate (IT)
(74) Representative: Borsano, Corrado

(57) **Abstract**

According to the invention a device is described for isolating contaminating agents comprising:
- a cabin for isolating contaminating agents with laminar flow (1), in turn comprising: a work chamber able to contain and allow the manipulation of contaminated objects, comprising an access opening to said work chamber from the outside; a generator of a vertical laminar filtered flow of air and a flow of air entering through the access opening;
- a decontamination station (2) connected to a first lateral wall of said work chamber through a first connecting opening, able to decontaminate an object to be decontaminated coming from said work chamber through said first connecting opening, and to transfer it to the outside through a third opening substantially decontaminated;
- a unit for transfer of contaminated material (3), removably connected to a second lateral wall of said work chamber through a second connecting opening, able to contain contaminated material coming from said work chamber through said second connecting opening, and move it to the outside for decontamination.

## Description

### Field of the invention.

The present invention concerns a system for handling contaminated bodies and materials, such as, for example, objects, substances and animals contaminated by viruses or other contaminating agents.

### Prior art

For the handling of objects contaminated by inorganic toxic substances or contaminating agents such as bacteria or viruses, devices called "isolators" or "glove boxes" are currently known.

Creating a physical barrier between the operator and the contaminating agents, these isolators ensure good operator safety and good isolation of the contaminating substances inside them. However, as the operator has to work with his arms inserted in sleeves or gloves fixed to the structure of the isolator, his movements are always awkward and limited.

Moreover, the transfer of material from the outside environment to the inside of the isolator and vice versa is a particularly critical operation.

So, depending on the type of objects or materials to be put into or taken out of the isolators, a great variety of introduction and/or extraction procedures and of devices and auxiliary equipment have been developed to accomplish them.

### Summary of the invention

The aim of the present invention is to supply a device and a method for isolating contaminated materials and objects which allow easier and faster handling of contaminated bodies, materials and objects than with isolators of a known type, and a faster transfer of objects and materials from and to the contaminated internal area of the device.

A first subject of the present invention is a device for isolating contaminating agents carried by contaminated bodies, objects or materials, characterised by comprising:
- a cabin for isolating contaminating agents with laminar flow, in turn comprising: a work chamber able to contain and allow the manipulation of said contaminated bodies, objects or materials, said work chamber comprising at least one access opening through which said work chamber is accessible from the outside; means for generating an air flow able to generate a vertical laminar filtered flow of air and an entering flow of air through said at least one access opening, able to avoid the exit of contaminating agents;
- a decontamination station connected to a first lateral wall of said work chamber through a first connecting opening, able to decontaminate said contaminated object coming from said work chamber through said first connecting opening, and to transfer it to the outside through a third opening substantially decontaminated;
- a unit for transfer of contaminated material, removably connected to a second lateral wall of said work chamber through a second connecting opening, able to contain said contaminated materials coming from said work chamber through said second connecting opening, and move it to the outside for decontamination.

A further subject of the present invention is a decontamination station for contaminated objects, characterised in that it comprises:
- a decontamination tank connected to a dividing wall between an infected environment and a not infected environment, through a third connecting opening, said decontamination tank being able to contain an object to be decontaminated coming from said work chamber, through said first connecting opening, and to contain a decontaminating liquid for said object;
- a fourth opening in said decontamination tank, from which said object is extracted after decontamination;
- a collecting tank for said decontaminating liquid;
- an hydraulic circuit for circulating said decontaminating liquid between said decontamination tank and said collecting tank making up disinfection cycles.

A still further subject of the present invention is a method for isolating contaminating agents carried by contaminated bodies, objects or materials, characterised in that it uses a device for isolating contaminating agents as described above, said method comprising the following steps:
- operating in said cabin for isolating contaminating agents with laminar flow for manipulating contaminated bodies, objects or materials;
- introducing a contaminated object hermetically closed in said decontamination station through said first connecting opening;
- activating a decontaminating cycle of said contaminated object hermetically closed in said decontamination station;
- at the end of said decontaminating cycle, extracting said object from said decontamination station substantially decontaminated;
- introducing contaminated material in said unit for transfer of contaminated material through said second connecting opening;
- removing said evacuation container to move it to a decontamination place.

These and further objects are achieved by means of an apparatus and method as described in the attached claims, which are considered an integral part of the present description.

### List of figures

The advantages that may be obtained with the present invention will be more clear, to a person skilled in the art, from the following detailed description of a particular embodiment of a non limiting character, with reference to the following figures, in which:

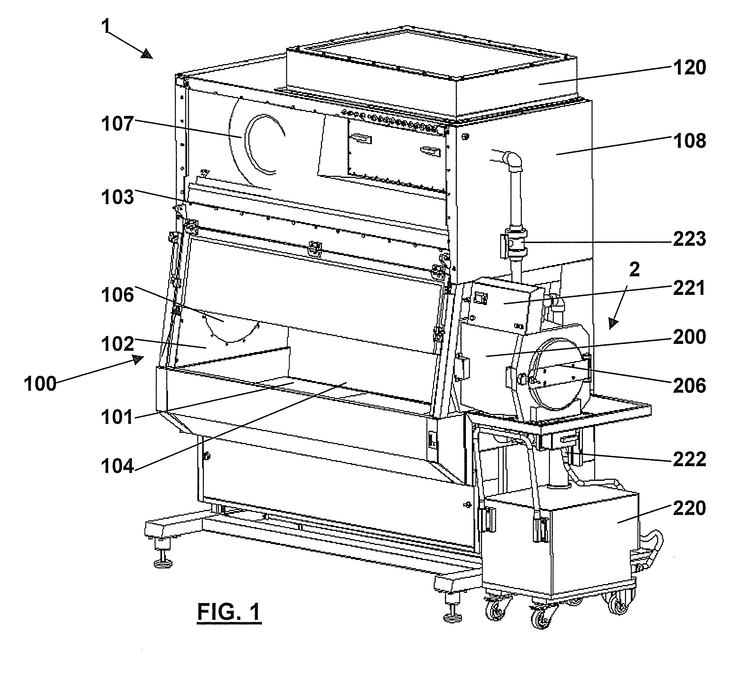

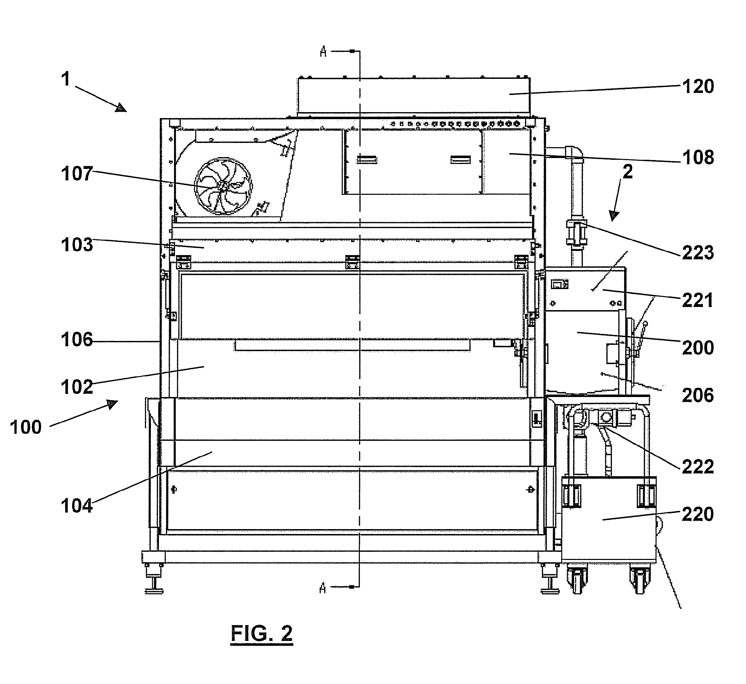

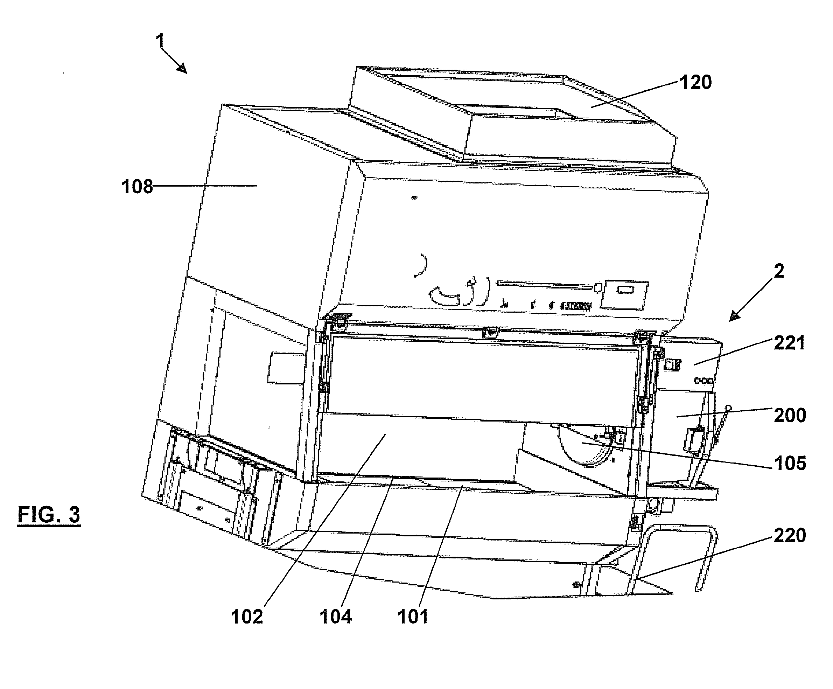

Figures 1, 2 and 3 schematically show front perspective views of a cabin for isolation and of a decontamination station being part of the device for isolating contaminated bodies and materials according to the present invention;

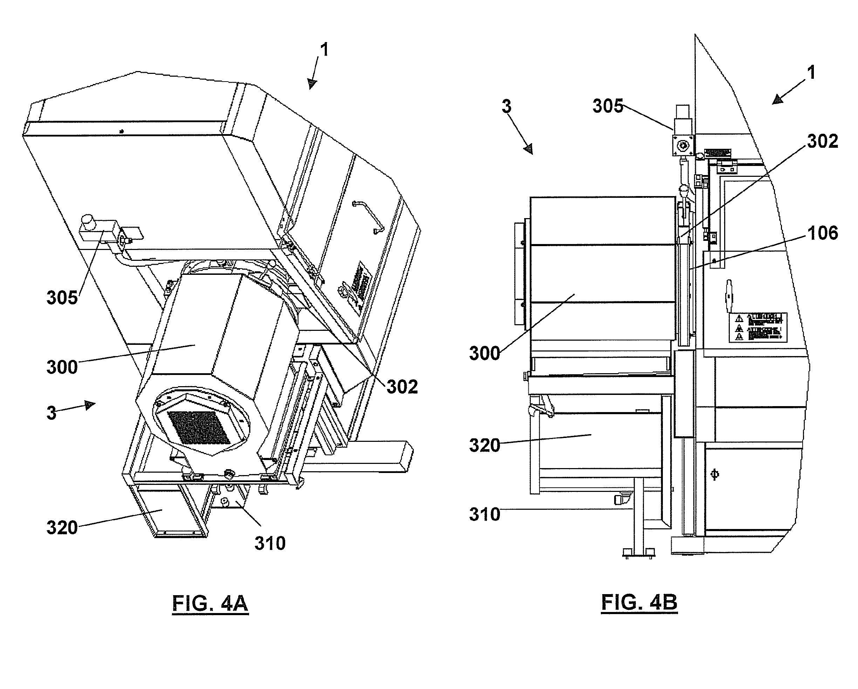

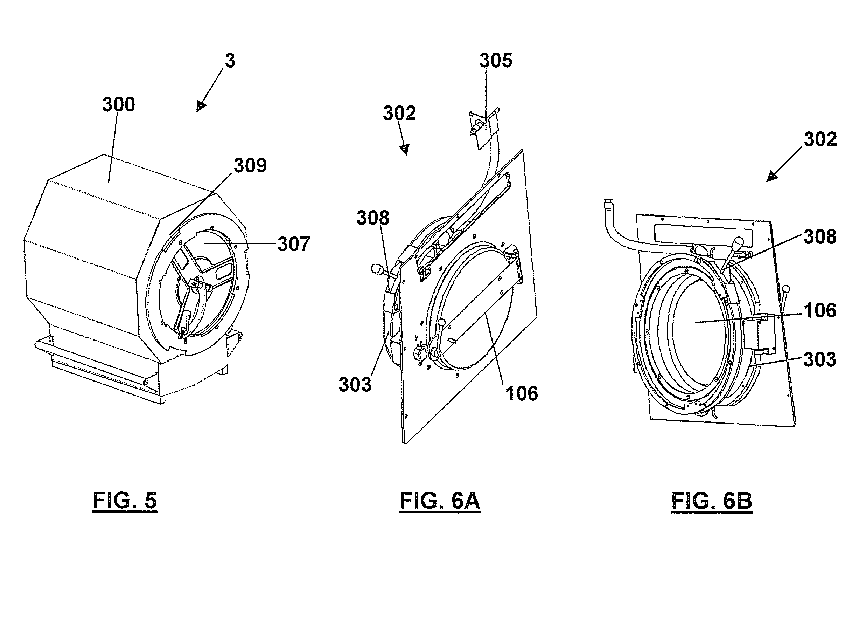

Figures 4A, 4B and 5 schematically show front perspective views of a unit for transfer of contaminated materials being part of the device for isolating contaminated bodies and materials according to the present invention;
Figures 6A and 6B schematically show an embodiment of a connection between the unit for transfer of contaminated materials and the cabin for isolation.

The same reference numerals in the figures designate the same elements.

### Detailed description

The Figures concern a preferred embodiment of a device for containing contaminating agents and for decontaminating objects according to the present invention.

This device comprises a cabin for isolation of contaminating agents with laminar flow 1, connected to a decontamination station 2, and to a unit for transfer of contaminated materials 3.

### Cabin for isolation of contaminating agents with laminar flow 1

The cabin 1 for isolation of contaminating agents (for example those classified at low and middle risk, suitable to be manipulated in cabins type BSC2) comprises a work chamber 100 defined by a plurality of side walls, a work surface 101 and a suitable filtering roof 108.

An operator can access the work chamber with his hands and arms from outside, through a front access opening 102.

A laminar air flow suitably purified and treated by an HEPA filter, is blown by a fan 107 through an air outlet 103 from the filtering roof 108, and sucked by suction outlets 104, present in the side bottom part of the work chamber 100, sidewise to the work surface 101. From the suction outlets 104 the air goes back to the filtering roof 108 through internal cavities not shown , and blown to the ambient through an air outlet 120 and filtered with an HEPA filter present in the air outlet 120 comprising an aspirator as well.

The air laminar flow prevents the contaminating agents (even inorganic toxic substances) present in the work chamber 100 from passing through the access opening 102 and being dispersed in the environment outside the isolating cabin 1. This way through the opening 102 there is an air inlet from the ambient in the working chamber 100. Advantageously, the laminar air flow is a descending flow situated approximately vertically.

The walls of the work chamber 100 present two connecting openings 105 and 106, for the connection by hermetic doors respectively to the decontamination station 2, and to the unit for transfer of contaminated materials 3. The opening 105 in the working chamber 100 is suitable to transfer a cage, externally contaminated, to the decontamination station 2, while the opening 106 id suitable to transfer to the outside of the working chamber 100 the contaminated materials, to be destroyed or decontaminated, toward the unit for transfer of contaminated materials 3.

The cabin for isolation 1 is particularly dedicated to the manipulation and cleaning of cages suited for housing laboratory animals, in the following called cages, of a known type, not shown in the figures.

The cages and the isolating cabin 1 have the aim of preventing exchanges of predetermined contaminating agents - for example viruses or bacteria - between the animals and the external environment and vice versa, protecting both the animals from pathogenic agents carried by the personnel external to the cages, and the laboratory personnel from the pathogenic agents carried by the animals.

The isolating cabin 1 in the present preferred embodiment is particularly dedicated to the cleaning and maintenance of cages with an hermetic closure, able to contain animals and materials, for example carriers of relatively dangerous contaminating agents, but to remain externally non contaminated, so that they can be manipulated by laboratory personnel operating in the premises with security level (Animal Biological Safety Level) ABSL1 or ABSL2 or ABSL3, and equipped, for example, with ordinary gowns, rubber or latex gloves, and a mask.

Such a cage may for example be taken from a housing and ventilation system of a known type, and inserted in the work chamber 100: there an operator can open it, clean it to remove excrement and residue of infected feed, fill it with fresh feed and close it again. Once closed, the cage must be disinfected on the outside or, more generally, decontaminated.

### Decontamination station 2

In the present preferred embodiment, the decontaminating station 2 comprises a so-called "dunk tank" system, that is a system of decontamination by immersion in a bath of decontaminating liquid, where the environment in which the object to be contaminated is immersed in the decontaminating bath is separate from the environment in which the object to be decontaminated is extracted from the decontaminating bath.

The decontaminating station 2 comprises essentially: a decontamination tank 200, connected on one side to the working chamber 100 through the opening 105, and yo the other side to the ambient through the opening 206; a collecting tank 220 containing a decontamination liquid, a control panel 221.

The openings 105 and 106 comprise interblocked hermetic doors.

A cage to be decontaminated of the hermetic closure type is introduced through the opening 105, and is inserted inside the decontamination tank 200, then closing the door of the opening 105. Then the decontaminating liquid is input in the decontamination tank 200 taking it from the below collecting tank 220 through an hydraulic circuit 222, and letting it flow to the inside of the tank 200. The inside of the tank 200 is filled by the decontaminating liquid, and the hermetic closure of the cage does not allow the liquid to penetrate inside the cage. A system 223 to allow the exit of the air from the insideof the tank 200 is also present, i.e. through suction, when the decontaminating liquid enters the tank. As the air is contaminated, it is brought to a filtering system, i.e. the system in the air outlet 120.

The cage is hermetically closed and therefore tends to float when surrounded by the decontaminating liquid, then in the tank 200 suitable locking means are provided holding the cage, resisting the hydrostatic thrust of the liquid.

After having remained immersed in the decontaminating liquid long enough to allow sufficient external decontamination of the cage, the decontaminating liquid is taken away from the inside of the tank 200, and reinserted in the collecting tank 220. The cage is then extracted from the decontamination tank 200, through the opening 206.

The collecting tank 220 is advantageously mounted on a trolley, so as it can be removed easily, i.e. for replacing the disinfecting liquid.

The hermetic doors of the openings 105 and 206 are interblocked, namely they can not be opened at the same time. The decontamination cycle starts after opening the door 105 and closing it again. Thereafter the door 206 opens. The procedure is controlled i.e. through the control panel 221 which may comprise an electronic control unit, and can be completely automatic, or it can provide for some manual control steps.

The decontamination station 2 can be used also independently from the rest of the system, as an autonomous station for the decontamination of the esternal part of objects, like the above cage, but more generally for hermetic containers, i.e. for laboratory samples. To the purpose the decontamination station 2 can be istalled at a dividing wall between two environments, the one infected and the other not infected. The door 105 will be in communication with the infected environment, while the door 206 with the not infected one.

### Unit for transfer of contaminated material 3.

The unit for transfer of contaminated material 3 comprises essentially: an evacuation container 300 connected to the opening 106 and communicating with the work chamber 100 through a closure system 302 described in the following; a supporting system 310 for supporting the evacuation container 300, especially when it is removed; a collecting tank 320 for containing decontaminating fluid, put in the supporting system 310; a trolley not shown for carrying the evacuation container 300, a double sealing cavity 303 between the two doors 106 and 307, which can be filled with decontaminating fluid. The side of the evacuation container 300 comprising the door 307 is fastened to the double sealing cavity 303 by a fastening mechanism closing suitable couplers 309 around the door 307.

The operator, when the unit for transfer of contaminated material 3 is connected to the cabin 1, first opens the door 106, then from the inside of the double sealing cavity 303 opens the door 307, which has a diameter sligtly smaller than that of the door 106 and can be opened towards the door 106 inside the cavity 303, inserts the contaminated material, and then closes both doors 307 and 106.

The internal side of the double sealing cavity 303, after removing the unit 3, would be in contact with the ambient, and that's why it must be decontaminated before removing the evacuation container 300. The decontaminating fluid is inserted in the cavity 303 through a blow-by hydraulic circuit 305, which takes it from the collecting tank 320, and puts it in the cavity 303 and lets it return in the tank 320.

At the end of the decontamination cycle, the evacuation container 300 can be removed by opening the fastening mechanism 308, and can be put on the trolley and carried to an handling system for the contaminated material. The opening of the fastening mechanism 308 is possible only at that time. The collecting tank 320 can be removed for changing the decontaminating fluid.

The above described procedure can be controlled automatically.

In a second embodiment, not shown in the figures, the closure system 302 comprises: the door 106 integral with the cabin 1; a sealing door, to give access to the inside of the evacuation container 300, which is in hermetic contact with the door 106, when the unit for transfer of contaminated material 3 is connected with the cabin 1; a flexible manifold in an autoclavable material, for the sealing connection between the sealing door and the evacuation container 300.

The sealing door is made in such a way that the external surfaces are never in contact with contaminated material.

When the door is opened when the unit for transfer of contaminated material 3 is connected with the cabin 1, the sealing door opens remaining integral with the door 106. After inserting the infected material in the evacuation container 300, the door 106 is closed. Now the consent is given for removing the evacuation container 300 and the sealing door is detached from the door 106, remaining integral with the container 300. The part of the sealing door which was in hermetic contact with the door 106 is now free in contact with the ambient. However it is never contaminated, as the two doors are in hermetic contact when the door 106 is opened, and therefore the sealing door does not need a decontamination.

### Operation of the complete system.

A preferred example is now described of use of the complete system described above, comprising the cabin 1, the decontamination station 2, and the unit 3.

An operator takes a cage not contaminated on the outside and situated in a non contaminated environment, but containing inside it for example a contaminated animal, and without needing to decontaminate it and/or further enclose it in other containers or bags decontaminated on the outside, he insert it in the work chamber 100 through the opening 102.

With respect to a known type of glove box, inserting in the laminar flow station can be carried out more quickly and conveniently.

Thanks to the effect of the entering air barrier at the front access opening 102, and to the laminar air flow, the introduction of the cage ensures the isolation of the contaminating agents inside the work chamber 100.

The operator in the contaminated zone opens the cage in the work chamber 100, and then closes it; then, through the opening 105 he puts the cleaned and closed cage in the decontamination tank 200, where it is decontaminated on the outside by immersing it in the decontaminating liquid, as descibed above. While through the opening 106 he transfers into the evacuation container 300 any materials to be destroyed in an incinerator such as excrement and remains of food contaminated by the animal, or also objects and materials to be sterilised in an autoclave - such as a contaminated empty cage, drinking troughs, etc. The operator closes the doors 106 and 307 and activates a suitable decontamination procedure, as described above, which decontaminate the double sealing cavity, if existing.

In case of use of double doors, as in the second embodiment described above, thanks to the hermetic sealing, the decontaminating system is not required.

After that the evacuation container 300 and the door 307, which closes it hermetically, are detached from the cabin 1, and are taken to an incinerator or to the autoclave, while the door 106 remains fixed on the cabin 1, keeping the infectious agents isolated in the chamber 100.

From what has been described it may be seen how, with a system according to the present invention, it is possible to manipulate contaminated objects and materials in an isolated contaminated environment, from which only sterilised materials and objects come out (for example the cages coming out of the decontamination station 2) or materials and objects closed in containers 300 that ensure a sufficient degree of isolation (for example even BSL3) and that may be manipulated with relatively low safety and isolation criteria (for example BSL1 or BSL2), also ensuring the complete safety of the operators.

The entire system allows a relatively large quantity of cages or other materials to be treated quickly.

The embodiments described above are susceptible to numerous modifications and variations, though without departing from the scope of the present invention. For example the decontamination tank 200 may be replaced with container of different types and shapes, or more generally with containing means suited to contain a bath of decontaminating liquid; objects different from the cages can be handled, more generally hermetic containers, i.e. for laboratory samples; the cages or other objects to be manipulated in the cabin 1 may be introduced into the work chamber 100 in various ways and with various inserting means, for example chutes or drops, conveyer belts, automatic or semiautomatic pushing, catching and pulling devices, etc.

Each modification and variation falling within the significance and field of equivalence of the claims is understood as being covered by them.

## Claims

1. Device for isolating contaminating agents carried by contaminated bodies, objects or materials, **characterised by** comprising:
- a cabin for isolating contaminating agents with laminar flow (1), in turn comprising:
- a work chamber (100) able to contain and allow the manipulation of said contaminated bodies, objects or materials, said work chamber comprising at least one access opening (102) through which said work chamber is accessible from the outside;
- means for generating an air flow (107, 103, 104) able to generate a vertical laminar filtered flow of air and an entering flow of air through said at least one access opening (102), able to avoid the exit of contaminating agents;
- a decontamination station (2) connected to a first lateral wall of said work chamber (100) through a first connecting opening (105), able to decontaminate said contaminated object coming from said work chamber (100) through said first connecting opening (105), and to transfer it to the outside through a third opening (206) substantially decontaminated;
- a unit for transfer of contaminated material (3), removably connected to a second lateral wall of said work chamber (100) through a second connecting opening (106), able to contain said contaminated materials coming from said work chamber (100) through said second connecting opening (106), and move it to the outside for decontamination.

2. Device for isolating contaminating agents according to claim 1, **characterised in that** said means for generating an air flow (107, 103, 104) are able to generate said vertical laminar filtered flow of air through one or more ait outlets (103) placed above said at least one access opening (102), towards one or more suction outlets (104), placed in the side bottom part of of said access opening (102) where said air flow is sucked together with said air flow coming in through said access opening (102), and carried through cavities internal to said cabin (1) and emitted to the ambient.

3. Device for isolating contaminating agents according to claim 1,
**characterised in that** it comprises inserting means suited for inserting said object in the work chamber (100) through said air flow coming in through said access opening (102).

4. Device for isolating contaminating agents according to claim 1, **characterised in that** said decontamination station (2) comprises:
- a decontamination tank (200) connected to said first lateral wall in said work chamber (100), able to contain an object to be decontaminated coming from said work chamber (100), through said first connecting opening (105), and to contain a decontaminating liquid for said object;
- a second opening (206) in said decontamination tank (200), from which said object is extracted after decontamination;
- a collecting tank (220) for said decontaminating liquid;
- an hydraulic circuit (222) for circulating said decontaminating liquid between said decontamination tank (200) and said collecting tank (220) making up decontamination cycles.

5. Device for isolating contaminating agents according to claim 4, **characterised in that** said first opening (105) and second opening (106) comprise interblocked hermetic-closure doors.

6. Device for isolating contaminating agents according to claim 5, **characterised in that** it further comprises control means (221) able to control the sequence of operations of said decontamination station (2) in such a way that:
- said interblocked hermetic-closure doors of said first opening (105) and second opening (206) do not open at the same time;
- after opening of said first opening (105), introducing said object to be decontaminated, and closing the door again, a decontamination cycle occurs, after which said second opening (106) can be opened, to let the object exit.

7. Device for isolating contaminating agents according to claim 4, **characterised in that** it further comprises an air suction system (223) to allow the air go out from the inside of said decontamination tank (200), when said decontaminating liquid is entering in, and that the air can be brought to a filtering system.

8. Device for isolating contaminating agents according to claim 4,
**characterised in that** said decontamination tank (200) comprises locking means for said object to be decontaminated.

9. Device for isolating contaminating agents according to claim 1, **characterised in that** said unit for transfer of contaminated material (3) comprises:
- an evacuation container (300) removably connected to said second connecting opening (106) through a closure system (302);
- a supporting system (310) for supporting said evacuation container (300), especially when it is removed;
- a collecting tank (320) for containing decontaminating fluid placed in said supporting system (310).

10. Device for isolating contaminating agents according to claim 9, **characterised in that** said closure system (302) comprises:
- a first hermetic-closure door in said second connecting opening (106);
- a second hermetic-closure door (307) for entrance to the inside of said evacuation container (300);
- a double sealing cavity (303) between said first and second door, which can be filled with decontaminating fluid;
- a fastening mechanism (308) for fastening the side of the evacuation container (300) comprising said second door (307) to said double sealing cavity (303);
- an hydraulic circuit (305) for circulating said decontaminating fluid between said evacuation container (300) and said collecting tank (320) after closure of said first and second hermetic-closure door, obtaing decontamination cycles, said fastening mechanism opening at the end of each decontamination cycle.

11. Device for isolating contaminating agents according to claim 9, **characterised in that** said closure system (302) comprises:
- a third hermetic-closure door in said second connecting opening (106);
- a fourth hermetic-closure door, for access to the inside of said evacuation container (300), with hermetic contact with said third hermetic-closure door when said evacuation container (300) is connected to said cabin (1), and opening hermetically together with said third hermetic-closure door;
- a flexible manifold in autoclavable material, for the hermetic connection between said fourth hermetic-closure door and said evacuation container (300), said flexible manifold detaching from said third hermetic-closure door and remaining integral with said evacuation container (300) when the latter is removed.

12. Device for isolating contaminating agents according to claim 9, **characterised in that** said unit for transfer of contaminated material (3) further comprises a trolley for carrying said evacuation container (300) to move it to the outside for decontamination.

13. Decontamination station (2) for contaminated objects, **characterised in that** it comprises:
- a decontamination tank (200) connected to a dividing wall between an infected environment and a not infected environment, through a third connecting opening, said decontamination tank (200) being able to contain said contaminated objects coming from said work chamber (100), through said first connecting opening (105), and to contain a decontaminating liquid for said object;
- a fourth opening in said decontamination tank (200), from which said object is extracted after decontamination;
- a collecting tank (220) for said decontaminating liquid;
- an hydraulic circuit (222) for circulating said decontaminating liquid between said decontamination tank (200) and said collecting tank (220) making up decontamination cycles.

14. Decontamination station (2) according to claim 13, **characterised in that** said third opening and said fourth opening (106) comprise interblocked hermetic-closure doors.

15. Decontamination station (2) according to claim 14, **characterised in that** it further comprises control means (221) able to control the sequence of operations of said decontamination station (2) in such a way that:
- said interblocked hermetic-closure doors of said third opening and fourth opening (206) do not open at the same time;
- after opening of said third opening, introducing said object to be disinfected, and closing the door again, a decontamination cycle occurs, after which said fourth opening (106) can be opened, to let the object exit.

16. Decontamination station (2) according to claim 13, **characterised in that** it further comprises an air suction system (223) to allow the air go out from the inside of said decontamination tank (200), when said decontaminating liquid is entering in, and that the air can be brought to a filtering system.

17. Decontamination station (2) according to claim 13, **characterised in that** said decontamination tank (200) comprises locking means for said object to be decontaminated.

18. Method for isolating contaminating agents carried by contaminated bodies, objects or materials, **characterised in that** it uses a device for isolating contaminating agents according to any of claims 1 to 12, said method comprising the following steps:
- operating in said cabin for isolating contaminating agents with laminar flow (1) for manipulating said contaminated bodies, objects or materials;
- introducing said contaminated object hermetically closed in said decontamination station (2) through said first connecting opening (105);
- activating a decontamination cycle of said contaminated object hermetically closed in said decontamination station (2);
- at the end of said decontamination cycle, extracting said object from said decontamination station (2) substantially decontaminated;
- introducing contaminated material in said unit for transfer of contaminated material (3) through said second connecting opening (106);
- removing said evacuation container (300) to move it to a decontamination place.
